# EUROPEAN PATENT SPECIFICATION

(11) **EP 3 467 120 B1**
(45) Date of publication and mention of the grant of the patent: **08.11.2023**
(21) Application number: 17802816.3
(22) Date of filing: 23.05.2017
(51) Int. Cl.: C12Q 1/60, C12Q 1/44, G01N 33/92

(54) **METHOD AND REAGENT FOR QUANTIFYING CHOLESTEROL IN TRIGLYCERIDE-RICH LIPOPROTEIN**
VERFAHREN UND REAGENZ ZUR QUANTIFIZIERUNG VON CHOLESTERIN IN TRIGLYCERIDREICHEM LIPOPROTEIN
PROCÉDÉ ET RÉACTIF POUR QUANTIFIER LE CHOLESTÉROL DANS LA LIPOPROTÉINE RICHE EN TRIGLYCÉRIDES

(30) Priority: 24.05.2016 JP 2016103108
(43) Date of publication of application: 10.04.2019
(73) Proprietor: Denka Company Limited, Tokyo 103-8338 (JP)
(72) Inventor: SATOH, Noriyuki, Gosen-shi Niigata 959-1834 (JP); IKAIDA, Makoto, Gosen-shi Niigata 959-1834 (JP); HIRAO, Yuhko, Gosen-shi Niigata 959-1834 (JP); ITOH, Yasuki, Gosen-shi Niigata 959-1834 (JP)
(74) Representative: Mewburn Ellis LLP
(86) International application number: PCT/JP2017/019279
(87) International publication number: WO 2017/204230

(56) References cited:
- EP-A1- 2 639 310
- EP-A2- 1 132 482
- WO-A1-2008/087895
- JP-A- 2001 124 780
- JP-A- 2008 141 975
- JP-A- 2016 034 252
- US-A1- 2009 023 167
- US-A1- 2009 170 139
- Anoymouus: "Cholesterol esterase [CEBP-M]", , XP002796644, Retrieved from the Internet: URL:http://www.asahi-kasei.co.jp/shindan/p df/T-98_catalog.pdf [retrieved on 2020-01-03]
- COHN, J.S. et al.: "Detection, quantification, and characterization of potentially atherogenic triglyceride-rich remnant lipoproteins", ARTERIOSCLER. THROMB. VASC. BIOL., vol. 19, no. 10, 1999, pages 2474-2486, XP055553523, ISSN: 1079-5642
- Cholesterol esterase from Schizophyllum commune, Retrieved from the Internet: URL:http://www.toyobo-global.com/ seihin/xr/enzyme/pdf_files/2010_037_040_CO E_ 301_302.pdf [retrieved on 2017-07-26]
- Cholesterol esterase from Pseudomonas sp.., Retrieved from the Internet: URL:http:// www.toyobo-global.com/seihin/xr/enzyme/pdf - files/2010_041_044_COE_311.pdf [retrieved on 2017-07-26]
- SVENDSEN, A. et al.: "Biochemical properties of cloned lipases from the Pseudomonas family", BIOCHIM. BIOPHYS. ACTA, vol. 1259, 26 October 1995 (1995-10-26), pages 9-17, XP055553527, ISSN: 0006-3002

## Description

### TECHNICAL FIELD

The present invention relates to a method and a reagent of quantifying cholesterol (hereinafter, when expressed as "lipoprotein name" cholesterol or "lipoprotein name"-C, it means cholesterol in the lipoprotein described in the quotation mark) in triglyceride-rich lipoprotein (hereinafter also referred to as "TRL").

### BACKGROUND ART

Lipoproteins present in blood are classified according to the difference in densities observed in ultracentrifugation into chylomicron, very-low-density lipoprotein (hereinafter also referred to as "VLDL"), intermediate-density lipoprotein (hereinafter also referred to as "IDL"), low-density lipoprotein (hereinafter also referred to as "LDL"), and high-density lipoprotein (hereinafter also referred to as "HDL"). It is known that these lipoproteins contain varying amounts of lipids and proteins, such as triglycerides and cholesterol, each exhibiting different actions *in vivo.*

TRL is a generic name for lipoproteins with a high content of triglycerides, including chylomicron, VLDL, intermediate metabolites thereof and remnant-like lipoprotein particles (RLP), and may also include IDL.

So far, involvement of LDL and RLP in arteriosclerotic disease is known. It has been reported that TRL including IDL is deeply involved in the development of arteriosclerotic disease, mainly in Europe and the United States, and has attracted attention.

Currently known TRL measurement methods include ultracentrifugation method and NMR method. Ultracentrifugation method is a method utilizing difference in the densities of the lipoproteins subjected to centrifugation, has disadvantages that it requires skill and days for work, and high cost. NMR method is a method of measuring the number of lipoprotein particles using magnetic resonance, needs special instruments and thus is not common.

Other methods of measuring TRL-C include a calculation method. Since TRL can also be considered as lipoproteins other than LDL and HDL, TRL-C level is calculated by subtracting levels of LDL-C and HDL-C from total level of cholesterol. As a method of measuring LDL-C, the β-Quantification method (BQ method) of the U.S. Centers for Disease Control is the international standard method. However, since this method considers lipoproteins having a density of 1.006 to 1.063 g/cm³ as LDL, LDL-C in the BQ method includes IDL-C. Therefore, when TRL-C is calculated by using LDL-C measured by BQ method, IDL-C is also subtracted as LDL-C. This also applies to other methods of measuring LDL-C that correlate with the BQ method, such as the Friedewald equation. When using these LDL-C measurement methods, since TRL-C calculated does not include IDL-C, TRL including IDL, which is said to be more clinically meaningful, is not measured.

Therefore, there is a need for a method and reagent for conveniently and accurately quantifying TRL-C including IDL to be invented.

Hereinafter, when simply described as TRL and TRL-C, they mean TRL including IDL and TRL-C including IDL-C, respectively.

There have been reported methods for measuring RLP-C, a part of TRL-C, in Patent Documents 1 to 4. All of the methods allow measurement using a general-purpose automatic analyzer, but since only RLP is measured in the methods, they are different from the present invention in which the entire TRL is measured.

### PRIOR ART REFERENCES

### PATENT DOCUMENTS

Patent Document 1: JP 4456715 B
Patent Document 2: JP 5027648 B
Patent Document 3: JP 5766426 B
Patent Document 4: US 2009/170139 A1

### SUMMARY OF THE INVENTION

### PROBLEMS TO BE SOLVED BY THE INVENTION

An object of the present invention is to provide a method of quantifying TRL-C in a test sample in a more specific manner without requiring laborious operations.

### MEANS FOR SOLVING THE PROBLEMS

The present inventors intensively studied to find that cholesterol esterase having a molecular weight of more than 50 kDa which is allowed to act on a test sample containing various types of lipoproteins in the presence of a surfactant(s) specifically reacts with lipoproteins other than TRL. The present inventors conceived that use of this finding allows convenient and accurate quantification of TRL-C without separation operation, thereby completing the present invention.

That is, the present invention provides the following.
[1] A method of quantifying cholesterol in triglyceride-rich lipoprotein (TRL-C), wherein the TRL-C is cholesterol in chylomicrons and remnants thereof, cholesterol in VLDL and remnants thereof, and cholesterol in IDL, the method comprising the steps of:
   (1) selectively eliminating cholesterol in lipoproteins other than triglyceride-rich lipoprotein (TRL) by allowing a cholesterol esterase having a molecular weight of more than 50 kDa as measured by SDS-polyacrylamide gel electrophoresis and a surfactant(s) to act; and
   (2) specifically quantifying the remaining TRL-C,
      wherein in cases where said surfactant used in said step (1) is one surfactant, said surfactant is one selected from the group consisting of polyoxyethylene polycyclic phenyl ether having an HLB value of 12 to 14; and
      wherein in cases where two or more surfactants are used, said surfactants comprise at least one selected from the group consisting of polyoxyethylene polycyclic phenyl ether, and the two or more surfactants are combined such that the combined surfactants have a total HLB value of 12 to 14.
[2] The method according to item [1], wherein the step (2) is carried out in the presence of a surfactant(s), and wherein the surfactant(s) comprise(s) at least one selected from the group consisting of polyoxyethylene alkyl ether, polyoxyethylene lauryl ether, lauryl alcohol alkoxylate, and polyoxyethylene polycyclic phenyl ether.
[3] The method according to item [2], wherein the polyoxyethylene polycyclic phenyl ether is at least one selected from the group consisting of polyoxyethylene styrenated phenyl ether.
[4] The method according to item [3], wherein the polyoxyethylene styrenated phenyl ether is at least one selected from the group consisting of polyoxyethylene distyrenated phenyl ether.
[5] The method according to any one of items [1] to [4], wherein the step (1) comprises allowing cholesterol esterase and cholesterol oxidase to act and then decomposing produced hydrogen peroxide; and wherein the step (2) comprises allowing cholesterol esterase and cholesterol oxidase to act and then quantifying produced hydrogen peroxide.
[6] Use of a kit in a method of quantifying cholesterol in triglyceride-rich lipoprotein TRL-C according to any one of items [1] to [5], the kit comprising the cholesterol esterase having a molecular weight of more than 50 kDa as measured by SDS-polyacrylamide gel electrophoresis and the surfactant(s) used in the step (1) of the method,
   wherein in cases where said surfactant used in said step (1) is one surfactant, said surfactant is one selected from the group consisting of polyoxyethylene polycyclic phenyl ether having an HLB value of 12 to 14; and
   wherein in cases where two or more surfactants are used, said surfactants comprise at least one selected from the group consisting of polyoxyethylene polycyclic phenyl ether, and the two or more surfactants are combined such that the combined surfactants have a total HLB value of 12 to 14.
[7] The use according to item [6], wherein the kit further comprises surfactant(s) for use in step (2) which surfactants is/are at least one selected from the group consisting of polyoxyethylene alkyl ether, polyoxyethylene lauryl ether, lauryl alcohol alkoxylate, and polyoxyethylene polycyclic phenyl ether.
[8] The use according to item [7], wherein the polyoxyethylene polycyclic phenyl ether is at least one selected from the group consisting of polyoxyethylene styrenated phenyl ether.
[9] The use according to item [8], wherein the polyoxyethylene styrenated phenyl ether is at least one selected from the group consisting of polyoxyethylene distyrenated phenyl ether.

### EFFECTS OF THE INVENTION

By the present invention, a novel method by which TRL-C in a test sample can be conveniently and accurately quantified with an automatic analyzer without requiring any separation operation, and use of a kit therefor are provided.

### BRIEF DESCRIPTION OF THE DRAWINGS

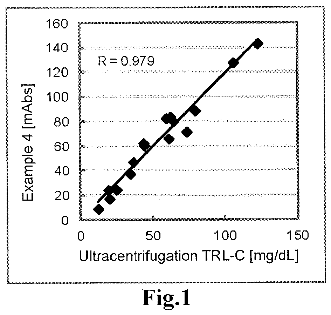

FIG. 1 shows a correlation between TRL-C determined by the method of the present invention described in Example 4 below and TRL-C determined by ultracentrifugation method.

### MODE FOR CARRYING OUT THE INVENTION

Cholesterol contained in lipoproteins includes esterified cholesterol (cholesterol ester) and free cholesterol. As used herein, the term "cholesterol" is simply used, the term includes both of the esterified cholesterol and the free cholesterol.

TRL-C to be quantified by the method of the present invention means cholesterol in lipoproteins which are chylomicron (including chylomicron remnant), VLDL (including VLDL remnant) and IDL, that is, cholesterol in lipoproteins having densities of less than 1.019 g/cm³ .

As the test sample to be subjected to the method of the present invention, any test sample containing TRL-C to be quantified can be used, and the test sample is usually a body fluid such as blood (including whole blood, serum and plasma) or a dilution thereof, but the test sample is not restricted thereto.

The term "reacting" to a lipoprotein as used herein means that the structure of the lipoprotein is changed by a surfactant and/or an enzyme, and an enzyme is likely to act on inner cholesterol.

In the step (1) in the present invention, cholesterol in lipoproteins other than TRL is specifically eliminated. Here, the term "eliminated" means decomposing cholesterol and to make the decomposed products undetectable in the subsequent step (2). An exemplary method of specifically eliminating cholesterol contained in lipoproteins other than TRL involves allowing cholesterol oxidase, a specific cholesterol esterase (described later) to act on a test sample and decomposing the produced hydrogen peroxide. Methods of decomposing hydrogen peroxide include, but are not limited to, a method of allowing catalase to act on hydrogen peroxide and decomposing it into water and oxygen; and a method of converting, for example, a hydrogen donor compound that produces a colorless quinone in response to hydrogen peroxide, into the colorless quinone by the action of peroxidase. The term "specifically eliminating" means that 90% or more, preferably 94% or more, more preferably 97% or more of cholesterol remaining after the eliminating is cholesterol in TRL. The percentage of cholesterol in TRL among the cholesterol remaining after the eliminating can be determined based on the correlation with measurement results obtained in ultracentrifugation method as described in detail in the Examples below. When the correlation is 0.9 or more, it is assumed that 90% or more of cholesterol remaining after the eliminating is cholesterol in TRL.

The method of specifically eliminating cholesterol in specific lipoproteins as in the first step is widely adopted, for example, in a method of determining LDL cholesterol (e.g., WO98/47005) and a method of determining HDL cholesterol (e.g., WO98/26090) and is well-known. The step (1) in the present invention can also be carried out in the same way as these well-known methods except that a particular cholesterol esterase described later is used.

Subsequently, in the step (2), cholesterol in TRL remaining without being eliminated in the above-described step (1) is enzymatically quantified. The method of enzymatically quantify cholesterol *per se* is well known in the art, including a method comprising allowing cholesterol oxidase and cholesterol esterase to act on cholesterol; converting produced hydrogen peroxide into quinone dye by peroxidase and hydrogen donor and hydrogen acceptor; and determining the absorbance of and quantifying the dye. This is a widely used and well-known method, and described as well in the above-mentioned WO98/47005 and WO98/26090.

When hydrogen peroxide produced in the step (1) is decomposed by catalase and the catalase is needed to be inhibited in the step (2), the catalase is inhibited by using a catalase inhibitor such as sodium azide in the step (2).

The most characteristic feature of the present invention resides in eliminating cholesterol in lipoproteins other than TRL with a cholesterol esterase having a molecular weight of more than 50 kDa in the presence of a surfactant. It is assumed that triglyceride present in TRL in a large amount together with esterified cholesterol sterically hinders a large molecular weight cholesterol esterase and prevents it from reaching and acting on esterified cholesterol inside particles, while a small molecular weight cholesterol esterase can penetrate into the inside of the particles and act.

The molecular weights of cholesterol esterase and subunits thereof are determined by conventional SDS-polyacrylamide gel electrophoresis (SDS-PAGE). As is well known, since SDS-PAGE is an electrophoresis method under reducing conditions, when the cholesterol esterase is composed of multiple subunits, the cholesterol esterase is decomposed into the subunits and thus the molecular weights of the distinct subunits are determined. On the other hand, when the cholesterol esterase has no subunit, the molecular weight of the cholesterol esterase is measured. Thus, the molecular weight of cholesterol esterase in the present invention is defined as the molecular weights of subunits when the cholesterol esterase has the subunits, or as the molecular weight of the cholesterol esterase when the cholesterol esterase does not have a subunit.

Cholesterol esterases having a molecular weight of more than 50 kDa are commercially available, for example, from Asahi Kasei Pharma and Kikkoman and can be used in the present invention. Since various cholesterol esterases having a molecular weight of 50 kDa or less are also commercially available, when commercially available products are used in the present invention, a cholesterol esterase having a molecular weight of more than 50 kDa has to be selected and used.

In the present invention, cholesterol esterase having a molecular weight of more than 50 kDa is used at least in the step (1) in the present invention. In the step (2), the cholesterol esterase used in step (1) may be directly used, or the same cholesterol esterase may be newly added, or cholesterol esterase having a different molecular weight may be added.

The steps (1) and (2) in the present invention involve at least one surfactant. Use of a suitable surfactant(s) can enhance the enzymatic reactivity in the steps.

The suitable surfactant(s) for the step (1) in the present invention include(s) at least one selected from the group consisting of polyoxyethylene polycyclic phenyl ether having an HLB value of 12 to 14.

Among the polyoxyethylene polycyclic phenyl ether, preferred is polyoxyethylene styrenated phenyl ether, and more preferred is polyoxyethylene distyrenated phenyl ether. However, any surfactant(s) selected from the group consisting of polyoxyethylene polycyclic phenyl ether having an HLB value of 12 to 14 can be used.

Specific examples of the surfactant that can be used in the step (1) in the present invention include Newcol 707, Newcol 708, Newcol 709, and Adekatol SP-12 (manufactured by ADEKA) as polyoxyethylene polycyclic phenyl ether; Blaunon DSP-12.5, Blaunon TSP-16 (both manufactured by Aoki Oil Industrial), and Noigen EA-137 (manufactured by DKS) as polyoxyethylene styrenated phenyl ether; and Emulgen A60 (manufactured by Kao) as polyoxyethylene distyrenated phenyl ether. These surfactants can be used alone or in combination of two or more.

The HLB value of the surfactant(s) used in the step (1) in the present invention is 12 to 14. One surfactant having an HLB value within the range from 12 to 14 may be used, or two or more surfactants each of which may have an HLB value outside the range from 12 to 14 may also be used as long as the surfactants are combined such that the total HLB value is within the range from 12 to 14. Although all of the above-described specific examples of the surfactant have HLB values within the range from 12 to 14, a surfactant(s) having an HLB value of 12 to 14 may or may not be included when two or more surfactant are combined, and surfactants that can be used in the step (1) in the present invention are not limited to the specific examples described above.

Suitable surfactants for the step (2) in the present invention preferably are those that react with all lipoproteins, including at least one selected from the group consisting of polyoxyethylene alkyl ether, polyoxyethylene lauryl ether, lauryl alcohol alkoxylate, and polyoxyethylene polycyclic phenyl ether. More specifically, examples of suitable surfactants include Emulgen 707, Emulgen 709, Emulgen 108 (all manufactured by Kao), Adekatol LB83, Adekatol LB103, Adekatol LB720 (all manufactured by ADEKA), Blaunon DSP-9 (manufactured by Aoki Oil Industrial), and Noigen EA-87 (manufactured by DKS).

The concentration of the surfactant used in the present invention in a reaction solution is preferably from 0.05% to 5%, more preferably from 0.1% to 1%, still more preferably from 0.1% to 0.75%. Note that as used herein, % means % by mass unless otherwise stated.

Any cholesterol oxidase can be used as long as the cholesterol oxidase is capable of oxidizing cholesterol to produce hydrogen peroxide in the present invention, including cholesterol oxidases derived from animals or microorganisms. The cholesterol oxidase may be one made by genetic engineering, with or without chemical modification.

In the step (1) in the present invention, a phospholipase may or may not be used. Specific examples of the phospholipase include, but are not limited to, phospholipase C (PLC), sphingomyelinase (SPC), phosphatidylinositol-specific phospholipase C (PI-PLC, all manufactured by Asahi Kasei Pharma); sphingomyelinase (derived from *Bacillus cereus*), sphingomyelinase (derived from *Staphylococcus aureus*), and phosphatidylinositol-specific phospholipase C (derived from *Bacillus cereus*, all manufactured by SIGMA). By using phospholipase, eliminating of cholesterol in lipoproteins other than TRL in the step (1) can be enhanced.

The amounts of enzymes used in the present invention, such as cholesterol esterase, cholesterol oxidase, and phospholipase, are not particularly limited and can be set as appropriate, usually from 0.001 to 2000 U/mL, preferably from 0.1 to 1000 U/mL.

Hydrogen donors used in the present invention is preferably an aniline derivative, and examples of the aniline derivative include N-ethyl-N-(2-hydroxy-3-sulfopropyl)-3-methylaniline (TOOS), N-ethyl-N-(2-hydroxy-3-sulfopropyl)-3,5-dimethyl aniline (MAOS), N-ethyl-N-(3-sulfopropyl)-3-methylaniline (TOPS), N-(2-hydroxy-3-sulfopropyl)-3,5-dimethoxyaniline (HDAOS), *N-*(3-sulfopropyl)aniline (HALPS), and *N*-(3-sulfopropyl)-3-methoxy-5-aniline (HMMPS).

As the hydrogen acceptor, 4-aminoantipyrine, methylbenzothiazolone hydrazone or the like can be used.

Each of the steps in the present invention is carried out preferably in a pH range of from 5 to 10, more preferably in a pH range of from 6 to 8.

Each of the steps is carried out preferably at a reaction temperature of from 2°C to 45°C, more preferably from 25°C to 40°C. Each of the steps is carried out preferably in a reaction time of from 1 to 10 minutes, more preferably from 3 to 7 minutes.

In carrying out the quantification method of the present invention, the reagent used may be divided into a plurality of reagent compositions. In the present invention, two reagent compositions can be prepared, e.g., one is a reagent composition for carrying out a step of eliminating cholesterol in lipoproteins other than TRL (*i.e*., step (1)) and another is a reagent composition for carrying out a step of measuring cholesterol in TRL (*i.e*., step (2)).

The reagent composition for carrying out the step (1) comprises at least a cholesterol esterase having a molecular weight of more than 50 kDa and the surfactant described above. The reagent composition may further contain cholesterol oxidase, a hydrogen donor such as an aniline derivative, and catalase for decomposing hydrogen peroxide.

The reagent composition for carrying out the step (2) comprises at least the surfactant described above. The reagent composition may further contain hydrogen acceptor such as 4-aminoantipyrine, and peroxidase.

To the reagent composition for carrying out the step (1) and the reagent composition for carrying out the step (2), monovalent cation (e.g., monovalent metal ion), divalent cation (e.g., divalent metal ion), or salts thereof, polyanion (e.g., heparin, dextran sulfate salt, phosphotungstic acid salt), or serum albumin, may be added, as necessary. Each reagent composition has a pH around neutral, for example, a pH of 5 to 9, preferably a pH of 6 to 8. The pH may be adjusted by adding a buffer.

Cholesterol in TRL may be quantified according to the method of the present invention, by adding a reagent composition for step (1) to a test sample and allowing the mixture to react; then adding a reagent composition for step (2) and allowing the mixture to react; and measuring the absorbance.

The present invention will now be described in detail with reference to examples, but is not limited thereto.

### EXAMPLES

### Example 1

Reagent composition A for step (1) (reagent compositions for step (1) also in Example 2 and subsequent examples are referred to as "reagent composition A") and reagent composition B for step (2) (reagent compositions for step (2) also in Example 2 and subsequent examples is referred to as "reagent composition B") were prepared as described below.

| Reagent composition A | |
|---|---|
| PIPES buffer, pH6.8 | 50 mmol/L |
| Various types of cholesterol esterase (see Table 1) | 3 U/mL |
| Cholesterol oxidase | 3 U/mL |
| Catalase | 1200 U/mL |
| TOOS | 2.0 mmol/L |
| Polyoxyethylene distyrenated phenyl ether [HLB: 12.8] | 0.25% (w/v) |

| Reagent composition B | |
|---|---|
| PIPES buffer, pH6.8 | 50 mmol/L |
| 4-aminoantipyrine | 4.0 mmol/L |
| Peroxidase | 20 Unit/mL |
| Sodium azide | 0.05% (w/v) |
| Polyoxyethylene alkyl ether | 0.5% (w/v) |

To 3 µL of a serum sample, was added 150 µL of reagent composition A, and the mixture was allowed to react for 5 minutes at 37°C. Next, 50 µL of reagent composition B was added and allowed to react for 5 min. Then, the absorbance at a main wavelength of 600 nm and a sub wavelength of 700 nm was measured. Table 1 shows the correlation coefficients as compared with the TRL-C concentration measured by the ultracentrifugation method as a comparative method.

**[Table 1]**

| Molecular weight of cholesterol esterase | Correlation coefficient |
|---|---|
| 62 kDa | 0.968 |
| 54 kDa | 0.968 |
| 29.5 kDa | 0.595 |

| | |
|---|---|
| (All of the cholesterol esterases are commercially available) | |

As shown in Table 1, use of cholesterol esterase exceeding 50 kDa in step (1) resulted in good correlation with the ultracentrifugation method.

### Example 2

Reagent composition A and reagent composition B were prepared as described below.

| Reagent composition A | |
|---|---|
| PIPES buffer, pH6.8 | 50 mmol/L |
| Cholesterol esterase [62 kDa] | 3 U/mL |
| Cholesterol oxidase | 3 U/mL |
| Catalase | 1200 U/mL |
| TOOS | 2.0 mmol/L |
| Various types of surfactant (see Table 2)* | 0.25% (w/v) |

| | |
|---|---|
| *When two or more surfactants are combined, the amount of 0.25% (w/v) is total. | |

| Reagent composition B | |
|---|---|
| PIPES buffer, pH6.8 | 50 mmol/L |
| 4-aminoantipyrine | 4.0 mmol/L |
| Peroxidase | 20 U/mL |
| Sodium azide | 0.05% (w/v) |
| Polyoxyethylene alkyl ether | 0.5% (w/v) |

To 3 µL of a serum sample, was added 150 µL of reagent composition A, and the mixture was allowed to react for 5 minutes at 37°C. Next, 50 µL of reagent composition B was added and allowed to react for 5 minutes. Then, the absorbance at main wavelength of 600 nm and sub wavelength of 700 nm was measured. Table 2 shows the correlation coefficients as compared with the TRL-C concentration measured by the ultracentrifugation method as a comparative method.

**[Table 2]**

| Surfactant | HLB | | Correlation coefficient |
|---|---|---|---|
| | alone | combination | |
| Polyoxyethylene polycyclic phenyl ether (alone) | 11.2 | - | 0.606 |
| | 12.3 | - | 0.941 |
| Polyoxyethylene styrenated phenyl ether (alone) | 10.6 | | 0.219 |
| | 13.0 | | 0.966 |
| | 14.3 | - | 0.187 |
| Polyoxyethylene distyrenated phenyl ether (alone) | 12.8 | - | 0.970 |
| | 14.5 | - | 0.080 |
| Polyoxyethylene styrenated phenyl ether (combination) | 10.6 | 12.8 | 0.917 |
| | 14.3 | | |
| Polyoxyethylene distyrenated phenyl ether (combination) | 12.8 | 13.5 | 0.947 |
| | 14.5 | | |

As shown in Table 2, use of a surfactant(s) selected from the group consisting of polyoxyethylene polycyclic phenyl ethers having an HLB value of 12 to 14 in step (1) resulted in good correlation with the ultracentrifugation method. Surfactants selected from the group consisting of polyoxyethylene polycyclic phenyl ethers having an HLB value other than from 12 to 14 which did not give good correlation results when used alone, gave good correlation results with ultracentrifugation when used in combination such that total HLB value was 12 to 14.

### Example 3

Reagent composition A and reagent composition B were prepared as described below.

### Reagent composition A

| | |
|---|---|
| PIPES buffer, pH6.8 | 50 mmol/L |
| Cholesterol esterase [62 kDa] | 3 U/mL |
| Cholesterol oxidase | 3 U/mL |
| Catalase | 1200 U/mL |
| TOOS | 2.0 mmol/L |
| Polyoxyethylene polycyclic phenyl ether [HLB: 12.8] | various concentrations (see Table 3) |

### Reagent composition B

| | |
|---|---|
| PIPES buffer, pH6.8 | 50 mmol/L |
| 4-aminoantipyrine | 4.0 mmol/L |
| Peroxidase | 20 Unit/mL |
| Sodium azide | 0.05 w/v% |
| Polyoxyethylene alkyl ether | 0.5 w/v% |

To 3 µL of a serum sample, was added 150 µL of reagent composition A, and the mixture was allowed to react for 5 minutes at 37°C. Next, 50 µL of reagent composition B was added and allowed to react for 5 minutes. Then, the absorbance at a main wavelength of 600 nm and a sub wavelength of 700 nm was measured. Table 3 shows the correlation coefficients as compared with the TRL-C concentration measured by the ultracentrifugation method as a comparative method.

**[Table 3]**

| Surfactant | HLB | Concentration (w/v%) | Correlation coefficient |
|---|---|---|---|
| Polyoxyethylene polycyclic phenyl | 12.8 | 0.1 | 0.956 |
| ether | | 0.2 | 0.946 |
| | | 0.25 | 0.961 |
| | | 0.3 | 0.971 |
| | | 0.4 | 0.979 |
| | | 0.5 | 0.977 |
| | | 0.75 | 0.924 |
| | | 1.0 | 0.801 |

As shown in Table 3, use of surfactant selected from the group consisting of polyoxyethylene polycyclic phenyl ethers in a concentration of 0.1 to 1.0 w/v% in step (1) resulted in good correlation with the ultracentrifugation method. In addition, when the concentration of the surfactant was 0.1 to 0.75 w/v%, better correlation with ultracentrifugation was shown.

### Example 4

Reagent composition A and reagent composition B were prepared as described below.

### Reagent composition A

| | |
|---|---|
| PIPES buffer, pH6.8 | 50 mmol/L |
| Cholesterol esterase [62 kDa] | 3 U/mL |
| Cholesterol oxidase | 3 U/mL |
| Catalase | 1200 U/mL |
| TOOS | 2.0 mmol/L |
| Polyoxyethylene distyrenated phenyl ether [HLB: 12.8] | 0.4% (w/v) |

### Reagent composition B

| | |
|---|---|
| PIPES buffer, pH6.8 | 50 mmol/L |
| 4-aminoantipyrine | 4.0 mmol/L |
| Peroxidase | 20 U/mL |
| Sodium azide | 0.05% (w/v) |
| Polyoxyethylene alkyl ether | 0.5% (w/v) |

To 3 µL of a serum sample, was added 150 µL of reagent composition A, and the mixture was allowed to react for 5 minutes at 37°C. Next, 50 µL of reagent composition B was added and allowed to react for 5 minutes. Then, the absorbance at main wavelength of 600 nm and sub wavelength of 700 nm was measured. FIG. 1 shows a correlation diagram as compared with the TRL-C concentration measured by the ultracentrifugation method as a comparative method.

As shown in FIG. 1, TRL-C measured according to the present invention showed good correlation with ultracentrifugation.

### Example 5

Reagent composition A and reagent composition B were prepared as described below.

### Reagent composition A

| | |
|---|---|
| PIPES buffer, pH6.8 | 50 mmol/L |
| Cholesterol esterase [62 kDa] | 3 U/mL |
| Cholesterol oxidase | 3 U/mL |
| Catalase | 1200 U/mL |
| TOOS | 2.0 mmol/L |
| Polyoxyethylene distyrenated phenyl ether [HLB: 12.8] | 0.25% (w/v) |

### Reagent composition B

| | |
|---|---|
| PIPES buffer, pH6.8 | 50 mmol/L |
| 4-aminoantipyrine | 4.0 mmol/L |
| Peroxidase | 20 Unit/mL |
| Sodium azide | 0.05% (w/v) |
| Various types of surfactant (see Table 4) | various concentrations (see Table 4) |

To 3 µL of a serum sample, was added 150 µL of reagent composition A, and the mixture was allowed to react for 5 minutes at 37°C. Next, 50 µL of reagent composition B was added and allowed to react for 5 minutes. Then, the absorbance at main wavelength of 600 nm and sub wavelength of 700 nm was measured. Table 4 shows the correlation coefficients compared with the TRL-C concentration measured by the ultracentrifugation method as a comparative method.

**[Table 4]**

| Surfactant | Concentration | Correlation coefficient |
|---|---|---|
| Polyoxyethylene alkyl ether | 0.5% (w/v) | 0.965 |
| | 0.5% (w/v) | 0.963 |
| Polyoxyethylene lauryl ether | 0.5% (w/v) | 0.966 |
| Lauryl alcohol alkoxylate | 0.5% (w/v) | 0.962 |
| | 0.5% (w/v) | 0.965 |
| Polyoxyethylene polycyclic phenyl ether | 0.5% (w/v) | 0.939 |
| | 1.0% (w/v) | 0.910 |

As shown in Table 4, use of a surfactant selected from the group consisting of polyoxyethylene alkyl ether, polyoxyethylene lauryl ether, and lauryl alcohol alkoxylate in step (2) resulted in good correlation with the ultracentrifugation method.

## Claims

1. A method of quantifying cholesterol in triglyceride-rich lipoprotein (TRL-C),
wherein the TRL-C is cholesterol in chylomicrons and remnants thereof, cholesterol in VLDL and remnants thereof, and cholesterol in IDL,
the method comprising the steps of:
(1) selectively eliminating cholesterol in lipoproteins other than triglyceride-rich lipoprotein (TRL) by allowing a cholesterol esterase having a molecular weight of more than 50 kDa as measured by SDS-polyacrylamide gel electrophoresis and a surfactant(s) to act; and
(2) quantifying the remaining TRL-C,
wherein in cases where said surfactant used in said step (1) is one surfactant, said surfactant is one selected from the group consisting of polyoxyethylene polycyclic phenyl ether having an HLB value of 12 to 14; and
wherein in cases where two or more surfactants are used, said surfactants comprise at least one selected from the group consisting of polyoxyethylene polycyclic phenyl ether, and the two or more surfactants are combined such that the combined surfactants have a total HLB value of 12 to 14.

2. The method according to claim 1, wherein said step (2) is carried out in the presence of a surfactant(s), and wherein said surfactant(s) comprise(s) at least one selected from the group consisting of polyoxyethylene alkyl ether, polyoxyethylene lauryl ether, lauryl alcohol alkoxylate, and polyoxyethylene polycyclic phenyl ether.

3. The method according to claim 2, wherein said polyoxyethylene polycyclic phenyl ether is at least one selected from the group consisting of polyoxyethylene styrenated phenyl ether.

4. The method according to claim 3, wherein said polyoxyethylene styrenated phenyl ether is at least one selected from the group consisting of polyoxyethylene distyrenated phenyl ether.

5. The method according to any one of claims 1 to 4, wherein said step (1) comprises allowing cholesterol esterase and cholesterol oxidase to act and then decomposing produced hydrogen peroxide; and wherein said step (2) comprises allowing cholesterol esterase and cholesterol oxidase to act and then quantifying produced hydrogen peroxide.

6. Use of a kit in the method of quantifying cholesterol in triglyceride-rich lipoprotein (TRL-C) according to any one of claims 1 to 5,
said kit comprising
(a) said cholesterol esterase having a molecular weight of more than 50 kDa as measured by SDS-polyacrylamide gel electrophoresis and
(b) said surfactant(s) used in said step (1) of the method,
wherein in cases where said surfactant used in said step (1) is one surfactant, said surfactant is one selected from the group consisting of polyoxyethylene polycyclic phenyl ether having an HLB value of 12 to 14; and
wherein in cases where two or more surfactants are used, said surfactants comprise at least one selected from the group consisting of polyoxyethylene polycyclic phenyl ether, and the two or more surfactants are combined such that the combined surfactants have a total HLB value of 12 to 14.

7. The use according to claim 6, wherein said kit further comprises surfactant(s) for use in step (2) which surfactants is/are at least one selected from the group consisting of polyoxyethylene alkyl ether, polyoxyethylene lauryl ether, lauryl alcohol alkoxylate, and polyoxyethylene polycyclic phenyl ether.

8. The use according to claim 7, wherein said polyoxyethylene polycyclic phenyl ether is at least one selected from the group consisting of polyoxyethylene styrenated phenyl ether.

9. The use according to claim 8, wherein said polyoxyethylene styrenated phenyl ether is at least one selected from the group consisting of polyoxyethylene distyrenated phenyl ether

## Patentansprüche

1. Verfahren zum Quantifizieren von Cholesterin in Triglyzerid-reichem Lipoprotein (TRL-C), wobei das TRL-C Cholesterin in Chylomikronen und Rückständen davon, Cholesterin in VLDL und Rückständen davon und Cholesterin in IDL ist, wobei das Verfahren die folgenden Schritte umfasst:
(1) selektives Eliminieren von Cholesterin in Lipoproteinen, die nicht Triglyzerid-reiches Lipoprotein (TRL) sind, durch Wirkenlassen einer Cholesterinesterase mit einem Molekulargewicht von mehr als 50 kDa, wie durch SDS-Polyacrylamidgelelektrophorese gemessen, und eines/von Tensids/Tensiden; und
(2) Quantifizieren des restlichen TRL-C;
wobei in Fällen, in denen das Tensid, das in Schritt (1) verwendet wird, ein Tensid ist, das Tensid eines ist, das aus der aus Polyoxyethylen-polyzyklisches-Phenyl-Ether mit einem HLB-Wert von 12 bis 14 bestehenden Gruppe ausgewählt ist; und
wobei in Fällen, in denen zwei oder mehr Tenside verwendet werden, die Tenside zumindest eines umfassen, das aus der aus Polyoxyethylen-polyzyklisches-Phenyl-Ether bestehenden Gruppe ausgewählt ist, und die zwei oder mehr Tenside so kombiniert werden, dass die kombinierten Tenside einen HLB-Gesamtwert von 12 bis 14 aufweisen.

2. Verfahren nach Anspruch 1, wobei Schritt (2) in Gegenwart eines/von Tensids/Tensiden durchgeführt wird und wobei das/die Tensid(e) zumindest eines umfasst/umfassen, das aus der aus Polyoxyethylenphenylether, Polyoxyethylenlaurylether, Laurylalkoholalkoxylat und Polyoxyethylen-polyzyklisches-Phenyl-Ether bestehenden Gruppe ausgewählt ist.

3. Verfahren nach Anspruch 2, wobei der Polyoxyethylen-polyzyklisches-Phenyl-Ether zumindest einer ist, der aus der aus Polyoxyethylen-styrolisiertes-Phenyl-Ether bestehenden Gruppe ausgewählt ist.

4. Verfahren nach Anspruch 3, wobei der Polyoxyethylen-styrolisiertes-Phenyl-Ether zumindest einer ist, der aus der aus Polyoxyethylen-distyrolisiertes-Phenyl-Ether bestehenden Gruppe ausgewählt ist.

5. Verfahren nach einem der Ansprüche 1 bis 4, wobei Schritt (1) das Wirkenlassen der Cholesterinesterase und Cholesterinoxidase und dann Abbauen von erzeugtem Wasserstoffperoxid umfasst; und wobei Schritt (2) das Wirkenlassen der Cholesterinesterase und der Cholesterinoxidase und dann das Quantifizieren des erzeugen Wasserstoffperoxids umfasst.

6. Verwendung eines Sets in dem Verfahren zum Quantifizieren von Cholesterin in Triglyzerid-reichem Lipoprotein (TRL-C) nach einem der Ansprüche 1 bis 5, wobei das Set Folgendes umfasst:
(a) die Cholesterinesterase, die ein Molekulargewicht von mehr als 50 kDa, wie durch SDS-Polyacrylamidgelelektrophorese gemessen, aufweist, und
(b) das/die Tensid(e), das/die in Schritt (1) des Verfahrens verwendet wird/werden,
wobei in Fällen, in denen das in Schritt (1) verwendete Tensid ein Tensid ist, das Tensid eines ist, das aus der aus Polyoxyethylen-polyzyklisches-Phenyl-Ether mit einem HLB-Wert von 12 bis 14 bestehenden Gruppe ausgewählt ist; und
wobei in Fällen, in denen zwei oder mehr Tenside verwendet werden, die Tenside zumindest eines umfassen, das aus der aus Polyoxyethylen-polyzyklisches-Phenyl-Ether bestehenden Gruppe ausgewählt ist, und die zwei oder mehr Tenside so kombiniert werden, dass die kombinierten Tenside einen HLB-Gesamtwert von 12 bis 14 aufweisen.

7. Verwendung nach Anspruch 6, wobei das Set weiters (ein) Tensid(e) zur Verwendung in Schritt (2) umfasst, wobei das/die Tensid(e) zumindest eines ist/sind, das aus der aus Polyoxyethylenalkylether, Polyoxyethylenlaurylether, Laurylalkoholalkoxylat und Polyoxyethylen-polyzyklisches-Phenyl-Ether bestehenden Gruppe ausgewählt ist.

8. Verwendung nach Anspruch 7, wobei der Polyoxyethylen-polyzyklisches-Phenyl-Ether zumindest einer ist, der aus der aus Polyoxyethylen-styrolisiertes-Phenyl-Ether bestehenden Gruppe ausgewählt ist.

9. Verwendung nach Anspruch 8, wobei der Polyoxyethylen-styrolisiertes-Phenyl-Ether zumindest einer ist, der aus der aus Polyoxyethylen-distyrolisiertes-Phenyl-Ether bestehenden Gruppe ausgewählt ist.

## Revendications

1. Procédé de quantification du cholestérol dans une lipoprotéine riche en triglycérides (TRL-C),
dans lequel la TRL-C est le cholestérol dans les chylomicrons et les restes de ceux-ci, le cholestérol dans les VLDL et les restes de ceux-ci, et le cholestérol dans les IDL, le procédé comprenant les étapes consistant à :
(1) éliminer sélectivement le cholestérol dans des lipoprotéines autres que la lipoprotéine riche en triglycérides (TRL) en permettant à une cholestérol estérase, présentant un poids moléculaire de plus de 50 kDa tel que mesuré par électrophorèse sur gel de SDS-polyacrylamide, et à un ou à des tensioactifs d'agir ; et
(2) quantifier la TRL-C restante,
dans lequel, dans des cas où ledit tensioactif utilisé dans ladite étape (1) est un certain tensioactif, ledit tensioactif est un certain sélectionné dans le groupe constitué par du phényléther polycyclique de polyoxyéthylène présentant une valeur HLB de 12 à 14 ; et
dans lequel, dans des cas où deux tensioactifs ou plus sont utilisés, lesdits tensioactifs comprennent au moins un certain sélectionné dans le groupe constitué par du phényléther polycyclique de polyoxyéthylène, et les deux tensioactifs ou plus sont combinés de telle sorte que les tensioactifs combinés présentent une valeur HLB totale de 12 à 14.

2. Procédé selon la revendication 1, dans lequel ladite étape (2) est effectuée en présence d'un ou de plusieurs tensioactifs, et dans lequel ledit ou lesdits tensioactifs comprennent au moins un sélectionné dans le groupe constitué par l'alkyléther de polyoxyéthylène, le lauryléther de polyoxyéthylène, un alcoxylate d'alcool laurylique et le phényléther polycyclique de polyoxyéthylène.

3. Procédé selon la revendication 2, dans lequel ledit phényléther polycyclique de polyoxyéthylène est au moins un sélectionné dans le groupe constitué par le phényléther styréné de polyoxyéthylène.

4. Procédé selon la revendication 3, dans lequel ledit phényléther styréné de polyoxyéthylène est au moins un sélectionné dans le groupe constitué par le phényléther distyréné de polyoxyéthylène.

5. Procédé selon l'une quelconque des revendications 1 à 4, dans lequel ladite étape (1) comprend une autorisation à la cholestérol estérase et à la cholestérol oxydase d'agir, puis une décomposition du peroxyde d'hydrogène produit ; et dans lequel ladite étape (2) comprend une autorisation à la cholestérol estérase et à la cholestérol oxydase d'agir, puis une quantification du peroxyde d'hydrogène produit.

6. Utilisation d'un kit dans le procédé de quantification du cholestérol dans une lipoprotéine riche en triglycérides (TRL-C) selon l'une quelconque des revendications 1 à 5, ledit kit comprenant
(a) ladite cholestérol estérase présentant un poids moléculaire de plus de 50 kDa tel que mesuré par électrophorèse sur gel de SDS-polyacrylamide et
(b) ledit ou lesdits tensioactifs utilisés dans ladite étape (1) du procédé,
dans lequel, dans des cas où ledit tensioactif utilisé dans ladite étape (1) est un certain tensioactif, ledit tensioactif est un certain sélectionné dans le groupe constitué par du phényléther polycyclique de polyoxyéthylène présentant une valeur HLB de 12 à 14 ; et
dans lequel, dans des cas où deux tensioactifs ou plus sont utilisés, lesdits tensioactifs comprennent au moins un certain sélectionné dans le groupe constitué par du phényléther polycyclique de polyoxyéthylène, et les deux tensioactifs ou plus sont combinés de telle sorte que les tensioactifs combinés préentent une valeur HLB totale de 12 à 14.

7. Utilisation selon la revendication 6, dans laquelle ledit kit comprend en outre un ou plusieurs tensioactifs à utiliser dans l'étape (2), lesquels tensioactifs sont au moins un choisi dans le groupe constitué par l'alkyléther de polyoxyéthylène, le lauryléther de polyoxyéthylène, l'alcoxylate d'alcool laurylique et le phényléther polycyclique de polyoxyéthylène.

8. utilisation selon la revendication 7, dans lequel ledit phényléther polycyclique de polyoxyéthylène est au moins un sélectionné dans le groupe constitué par le phényléther styréné de polyoxyéthylène.

9. utilisation selon la revendication 8, dans lequel ledit phényléther styréné de polyoxyéthylène est au moins un sélectionné dans le groupe constitué par le phényléther distyréné de polyoxyéthylène.
